# EUROPEAN PATENT APPLICATION

(11) **EP 2 163 880 A1**
(43) Date of publication of application: **17.03.2010**
(21) Application number: 08164070.8
(22) Date of filing: 10.09.2008
(51) Int. Cl.: G01N 1/34, B01L 3/00, G01N 33/569

(54) **Method and use for the separation of biological material from a sample fluid**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention provides a method and a device for the separation of biological material, like bacteria, from a sample fluid using chemotaxis of the biological material. The biological material is transferred from a sample fluid to a receiving fluid.

## Description

### FIELD OF THE INVENTION

The present invention relates to a method, a device and the use of a microfluidic device for the separation of biological material from a sample fluid.

### BACKGROUND OF THE INVENTION

Bacterial culturing and exposure to antibiotics has for many years been the standard for determining the strain of bacteria causing a bacterial infection and its susceptibility to anti-bacterial drugs. While this approach is very powerful and versatile, it is a time consuming process taking 1-3 days to complete. For some infections, particularly those contracted by immuno-compromised patients in intensive care, this delay can prove fatal. Medics are aware of this and often start broad-spectrum antibiotic cocktails before bacterial identification has been carried out and the antibiotic resistance characteristics are determined. This empirical strategy, however, fails in 20-40% of cases and the unnecessary use of broad-spectrum antibiotics only causes further bacterial resistance.

There are two alternatives for traditional culturing of bacterial samples which in principle can be carried out in much shorter periods of time. These are: (i) genomic analysis of bacteria and (ii) rapid culturing of single bacteria. In both these cases it is necessary, or at least preferable, to isolate the bacteria from the human cells present in the sample liquid. For genomic identification this prevents false positives from human DNA, which could interfere with the PCR-reaction for copy replication. Rapid culturing of single bacteria is based on immobilizing single bacteria on a surface and monitoring their response over time. Since the number of pathogens is in the order of 10-100 bacteria per milliliter of blood (compared to about 5 billion red blood cells, 300 million platelets, and 7 million white blood cells) it is clear that single cell analysis requires separation of bacteria from human cells. Up-concentration of bacteria is also suitable to reduce the volume of sample to be processed.

Whether genomic methods or rapid bacterial culturing is chosen as an alternative to conventional culturing, it is always preferable to perform up-concentration of the bacteria in the patient sample before identification and susceptibility testing. Currently, this is done using conventional bench-top methods such as filtration and centrifugation. These methods are very difficult to integrate in lab-on-chip devices, which are increasingly being used both for genomic analysis and rapid culturing of single bacteria. Lab-on-a-chip technologies are advantageous compared to traditional techniques, since the entire analysis chain can be integrated on an automated chip. Small sample volumes and chips result in very fast and cheap assays.

It is also highly preferable that the bacteria are viable specimens. Although it is known that cultures suffer from many false negative results, they are considered the standard. Other tests are disadvantageous for the fact that they can not distinguish between dead and living pathogens.

US 2005/0148064 A1 discloses a microfluidic device for fractionating and/or trapping selected molecules with a diffusion barrier or porous membrane. Target molecules are immobilized on or separated by a membrane having specific properties in relation to the target molecules.

### SUMMARY OF THE INVENTION

It is an object of the present invention to provide a method and a device for the separation of biological material from a sample which method can easily be performed in a lab-on-chip device. A further object of the present invention is to provide a method that is suitable for separating biological material based on the viability of the biological material.

These and other objects are solved by the features of the independent claims.

The present invention is directed to a method and a use of a microfluidic device for the separation of biological material from a sample fluid.

A first aspect of the invention provides a method for the separation of biological material from a sample fluid comprising the steps of
- providing a sample fluid, wherein the sample fluid contains biological material;
- providing a receiving fluid;
- allowing an exchange between the sample fluid and the receiving fluid at a contact area; and
- collecting the receiving fluid containing separated biological material, wherein the sample fluid and/or the receiving fluid contains a chemotactic substance inducing chemotaxis in the biological material.

In a preferred embodiment of the first aspect of the invention, the contact area between the sample fluid and the receiving fluid is formed by a porous or semi-permeable membrane, preferably a membrane being permeable to the biological material.

In another preferred embodiment of the first aspect of the invention, the sample fluid and the receiving fluid are in direct contact with each other.

In yet another preferred embodiment of the first aspect of the invention, the biological material is selected from the group consisting of bacteria, archea and eukaryotes. Examples of these are specific bacteria, protozoa, fungi, algae and mammalian cells, especially human cells.

In a further preferred embodiment of the first aspect of the invention, the sample fluid contains a chemotactic substance, wherein the chemotactic substance is a chemorepellent.

In another preferred embodiment of the first aspect of the invention, the receiving fluid contains a chemotactic substance, wherein said chemotactic substance is a chemoattractant.

Yet another preferred embodiment of the first aspect of the invention is directed to a method, wherein at least one of the sample fluid and the receiving fluid is flowing as a continuous flow, wherein the direction of flow of the sample fluid and the direction of flow of the receiving fluid preferably are parallel or anti-parallel to each other. It is especially preferred that the flow of the sample fluid and/or the flow of the receiving fluid is laminar or substantially laminar.

In still another preferred embodiment of the first aspect of the invention, at least one electrode is provided at the contact area or close to the contact area.

A second aspect of the invention is directed to the use of a microfluidic device for the separation of biological material from a sample fluid.

A third aspect of the invention provides a device for the separation of biological material comprising:
- at least two compartments;
- at least one membrane separating said at least two compartments;
- at least two electrodes being located close to the at least one membrane; and optionally one pre-exisiting chemical gradient.

### BRIEF DESCRIPTION OF THE DRAWINGS

Fig. 1 shows a first embodiment of the invention according to Example 1.
Fig. 2 shows a second embodiment of the invention according to Example 2.
Fig. 3 shows a third embodiment of the invention according to Example 3.
Fig. 4a shows a fourth embodiment of the invention according to Example 4.
Fig. 4b shows a preferred embodiment of the fourth embodiment.
Fig. 5 shows a fifth embodiment of the invention according to Example 5.

### DETAILED DESCRIPTION OF EMBODIMENTS

Before the invention will be described in detail with respect to some of its preferred embodiments, the following general definitions are provided.

As used in this specification and in the appended claims, the singular forms of "a" and "an" also include the respective plurals unless the context clearly dictates otherwise.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group, which preferably consists only of these embodiments.

Further definitions of terms will be given in the context of which the terms are used.

The present invention is directed to a method for the separation of biological material from a sample fluid. The method for separation uses an effect known as chemotaxis. The present invention is also directed to a device for the separation of biological material. The following embodiments apply to both the method and the device.

Chemotaxis is the phenomenon in which biological materials, like bodily cells, bacteria, and other single-cell or multicellular organisms, direct their movements according to certain chemical substances in their environment. The presence of a chemical substance can induce a movement of the biological material in a specific direction, depending on a gradient of concentration of the chemical substance in the surrounding medium. The chemical substance has a chemotactic property in relation to the biological material. Chemotaxis is called positive if movement is in the direction of a higher concentration of the chemical substance in question, and negative if the direction is opposite. Chemical substances inducing positive chemotaxis are called chemoattractants, and substances inducing negative chemotaxis are called chemorepellents.

Not only eukaryotic cells can move in the direction of a chemoattractant but other organisms, such as bacteria, can also displace themselves in a pre-determined direction. Unlike eukaryotic cells, bacteria do not have to adhere to a surface in order to develop traction and instead often use flagella or cilia to displace themselves in the direction of a chemical gradient. The speed of bacteria can vary enormously. In general, eukaryotic cells which require a surface for traction (i.e. all blood cells) can move between 0.3 to 11 µm/s. Flagellated bacteria can swim at speeds from 20-200 µm/s, and ciliated bacteria can push themselves to speeds as high as 400-2000 µm/s.

This chemotaxis can be used to separate biological material in a sample fluid from other undesired material in the sample fluid. The separation can be based on several different properties.

At first, the effect of chemotaxis can be different for different biological species. Some species might not react on a specific chemical substance, while others develop a strong chemotaxis in the presence of said specific chemical substance. This difference in chemotactic reaction can be used according to the present invention to differenciate between different species.

Secondly, different biological species might have different mechanisms of movement resulting in different speeds. Again, said difference in speed can be used according to the present invention to separate different biological species.

Finally, some biological species might not be susceptible for chemotaxis at all, as for example non viable species. It is thus possible to separate viable from non viable species by the method according to the present invention.

The separation can even include viable specimen which are not able to divide any more, but which are still pathogenic. Such viable specimen would result in a negative culturing, but can still be separated and analysed by the method of the present invention.

Accordingly, in a preferred embodiment, a chemically repellent chemotactic substance is present in the sample fluid, inducing a negative chemotaxis within the sample fluid driving the desired biological material to the receiving fluid. A similar effect can be achieved with a chemically attracting chemotactic substance in the receiving fluid inducing a positive chemotaxis towards the receiving fluid, drawing the desired biological material towards the receiving fluid. Furthermore, it is also possible to use both effects, i.e. to have a chemorepellent in the sample fluid and a chemoattractant in the receiving fluid at the same time.

The inventive device which comprises at least two compartments, at least one membrane separating said at least two compartments as well as at least two electrodes being located close to the at least one membrane. It will be understood by the skilled person that the term "at least two electrodes being located close to the at least one membrane" describes the arrangement of the electrodes in the vicinity of the membrane or even inside the membrane so that that the desired blocking or electrophoresis effect is achieved. Further details in this context are described below.

The electrodes in the inventive device serve to create a field barrier which blocks or prevents certain bioparticles from passing through the membrane. Therefore, the electrodes must be located sufficiently close to the membrane or the openings of the membrane. This can be achieved by partly or fully integrating the electrodes into the membrane or by arranging the electrodes in the vicinity of the membrane. In the latter case, a suitable distance between electrodes and membrane depends e.g. on the size of the membrane openings or membrane channels and the distance between the openings. The skilled person will have no difficulties in establishing a suitable distance which often may be below 50µm. According to an especially preferred embodiment, the at least two electrodes are situated or arranged parallel to the at least one membrane.

According to one preferred embodiment, the inventive device comprises one pre-exisiting chemical gradient. A "pre-existing chemical gradient" in the meaning of the present invention refers to an amount of a chemotactic substance which is able to induce chemotaxis in the biological material and which is contained in the device prior to adding the sample fluid or carrying out the inventive separation method. The at least one pre-exisiting chemical gradient can be achieved by providing the inventive device with an amount of chemoattractant and/or chemorepellent being sufficient for carrying out the separation method of the present invention.

According to another preferred embodiment of the inventive device, the chemotactic substance, i.e. the chemoattractant and/or chemorepellent is contained within one compartment of the device in the form of a solution, a powder or as a coating. The device being e.g. coated with the chemotactic substance has a pre-existing chemical gradient and can be provided in a ready-to-use embodiment. In another embodiment of the inventive device, at least one compartment comprises a chemoattractant in the form of a solution, a powder or as a coating and at least one other compartment comprises a chemorepellent in the form of a solution, a powder or as a coating. It is especially preferred to coat the inventive device with the chemotactic substance, i.e. the chemoattractant and/or chemorepellent in order to provide the pre-existing chemical gradient.

The pre-existing chemical gradient or the chemotactic substance(s) can be applied to the device by filling at least one solution containing the chemoattractant and/or chemorepellent into at least one of the compartments or by filling at least one powder, e.g. a freeze-dried powder or a carrier substance containing the chemoattractant and/or chemorepellent and being in the form of a powder or solid substance into at least one of the compartments. Alternatively, at least one of the compartments may be fully or partly, i.e. locally coated with t least one the chemoattractant and/or chemorepellent , so that the at least one chemoattractant and/or chemorepellent is solved upon addition of the sample fluid and/or the receiving fluid so that the inventive separation method can be carried out. Any combination of the foregoing embodiments is also contemplated according to the present invention (one compartment may be coated, whereas the other compartment may contain a powder).

The chemotactic substance, i.e. the chemoattractant and/or the chemorepellent, can be specific to a single biological material, or it can be a general chemoattractant or chemorepellent being effective for a wide variety of biological species. Preferably, the chemotactic substance is selected from the group of sugars, like glucose; growth factors, like VEGF; chemokines, like interleukin-8; phenol and phenol derivatives; and alcohols and polyhydric alcohols, like glycerol.

The chemoattractant may be selected from sugars, growth factors, and chemokines, more preferably from aspartate, arginin, N-acetylglucosamine, glucosamine, glucosamine dimers (chitosan), glutamate, and glucose.

The chemorepellent may be selected from phenol and phenol derivatives; and alcohols and polyhydric alcohols.

As an example, aspartate is an chemoattractant for wild type *E. coli* RP437, whereas glycerol is a chemorepellent. Similarly, *Borrelia burgdorferi* is chemoattracted by N-acetylglucosamine, glucosamine, glucosamine dimers (chitosan), glutamate, and glucose. As another example, *Pseudomonas aeruginosa* is attracted by e.g. arginin, and *Salmonella typhimurium* is attracted by aspartate.

Generally, all biological material showing chemotaxis can be separated from a sample fluid with the method according to the present invention. Preferably, the biological material to be separated is selected from the group consisting of eukaryotic cells, bacteria, fungi, algae, and protozoa, or other pathogens. Specifically preferred biological material are bacteria.

The bacteria may be selected from genera such as Bacillus, Vibrio, Escherichia, Shigella, Salmonella, Mycobacterium, Clostridium, Cornyebacterium, Streptococcus, Staphylococcus, Haemophilus, Neisseria, Yersinia, Pseudomonas, Chlamydia, Bordetella, Treponema, Stenotrophomonas, Acinetobacter, Enterobacter, Klebsiella, Proteus, Serratia, Citrobacter, Enterococcus, Legionella, Mycoplasma, Chlamydophila, Moraxella, and Morganella. Similarly, the fungi may be selected from genera such as Candida, and Aspergillus.

The bacteria may also be defined according to their reactivity, and may be selected from the group of gram-positive cocci, gram-positive bacilli, gram-negative cocci, gram-negative bacilli, or bacteria which are not possible to gram stain. Examples of gram-positive bacilli are Clostridium difficile, Bacillus anthracis or Bacillus cereus. Gram-positive cocci can be e.g. Staphylococcus aureus, Streptococcus pneumoniae or Staphylococcus epidermidis. Gram-negative bacilli are e.g. Escherichia coli, Legionella pneumophila or Pseudomonas aeruginosa. Gram-negative cocci are e.g. Neisseria gonorrhoeae, Neisseria meningitides or Moraxella catarrhalis.

The biological material or biological species to be separated is present in a sample fluid. While performing the method of the present invention, at least some of the biological material is separated from the sample fluid and collected within the receiving fluid. Preferably, at least 50 % of the biological material is collected within the receiving fluid, more preferably at least 80 %, even more preferably at least 95 %, further preferably at least 98 %, and most preferably at least 99,5 %, based on the number count of biological material.

The separation of the biological material is preferably performed in a time period from a few seconds to several hours, preferably from 60 seconds to 120 minutes, further preferably from 60 seconds to 30 minutes. The chemotactic rate or separation process time depends, inter alia, on the type of bacteria. The inventive method is especially suitable for bacteria showing a high chemotactic speed, e.g. of approx. 100 µm/s.

The biological material to be separated according to the method of the invention is part of a sample fluid. The sample fluid may contain a single biological material, or several different species of biological material. Typical sample fluids according to the present invention are body fluids, like blood, saliva and urine samples. However, all other bodily fluids can also be used according to the present invention. Additionally, the invention can also be used for environmental sample fluids, like sample fluids of lakes and rivers, or food sample fluids, like milk and juices. If a liquid sample fluid is not readily available, the biological material present, like bacteria, can also be brought into liquid form by washing the biological material from a surface, or by providing a smear from a biological material containing surface or collecting bacteria from isolated colonies on an agar plate and suspending the collected biological material in a sample fluid, like an electrolyte. Thus, the sample fluid may further contain an electrolyte, or a pH buffer, like phosphate buffered saline (PBS), 4-(2-hydroxyethyl)-1-piperazine ethanesulfonic acid (HEPES), and tris(hydroxymethyl)aminomethane (TRIS).

A sample fluid in accordance with the present invention is a fluid containing biological material. The biological material to be separated is suspended within the sample fluid. Preferably, the sample fluid is based on water. It is also encompassed within the present invention that the sample fluid contains other substances and material, like salts or cells, which might not be affected by the method according to the present invention, and which are tolerated by the method of the present invention.

Similarly, the receiving fluid can be any fluid suitable for the present method. Preferably, the receiving fluid is a liquid, like water or an alcohol. Also, the receiving fluid can contain additional compounds or material, apart from a possibly present chemoattractant, like growth media for bacteria, a lysing agent when the receiving fluid is to be genetically tested, or salts to specifically control the liquid conductivity. The latter can be particularly useful if barrier electrodes are to be used.

The sample fluid and the receiving fluid are contacted at a contact area allowing an exchange between the sample fluid and the receiving fluid. The contact area may be a membrane, preferably a porous or semi-permeable membrane, especially a membrane being permeable to the biological material to be separated. The membrane material to be used for the inventive method or the inventive device may be, for example, a polycarbonate, a polyester, a polyacrylamide, nitrous cellulose or agar. In a specifically preferred embodiment, the membrane is made from a photoresist material, like SU8. Even more preferably, the membrane is also permeable to the chemotactic substance.

The contact area may also be the area of the flow of the sample fluid and the flow of the receiving fluid. Preferably, both the sample fluid and the receiving fluid have a laminar flow behavior, i.e. the fluids result in a laminar flow. With a laminar flow, the mixing between the sample fluid and the receiving fluid is very low, or even not present at all. The exchange of material, or the transport of material, from the sample fluid into the receiving fluid is due to diffusion at the interface or contact area. It is thus possible to have a flow of sample fluid and a flow of receiving fluid with a contact area or interface between the two fluids without substantial mixing of the fluids. However, a chemoattractant may diffuse slowly from the receiving fluid into the sample fluid creating a gradient between the two flows, and the biological material is able to cross the contact area into the receiving fluid.

A flow of the sample fluid and the receiving fluid is not necessary for the method of the present invention. However, it is possible that either or both fluids are flowing. The flow can be parallel to each other, i.e. both fluids flow in the same direction, or anti-parallel to each other, i.e. both fluids flow in opposite directions. It is also possible that the two fluids come in contact to each other at a crossing of two flows.

The movement of the biological material through a contact area, especially a porous membrane, can be supported by a pressure difference between the pressure in the sample fluid and the pressure in the receiving fluid, i.e. the pressure in the sample fluid differs from the pressure in the receiving fluid. If the pressure of the sample fluid is slightly higher than the pressure of the receiving fluid, the passing of biological material through the contact area can be supported. On the other hand, a lower pressure of the sample fluid in comparison to the receiving fluid can limit the chemotactic effect. A pressure difference can be achieved with different flow speeds of the sample fluid and the receiving fluid.

The separation of biological material from a sample fluid can also be affected by the integration of electrodes to aid the separation of biological material. Electrodes placed close to the contact area work via the effect of dielectrophoresis.

According to the present invention, a device for the separation of biological material is provided which uses the effect of dielectrophoresis in addition to chemotaxis and which comprises at least two compartments, at least one membrane separating said at least two compartments, at least two electrodes and, optionally a pre-existing chemical gradient. The compartments are also referred to as volumes within this application. The compartments hold the sample fluid and the receiving fluid, separating both fluids by a membrane.

When an AC electrical field is applied to the electrodes then a high electrical field region is created between them. With an electrical field in the sample fluid, some biological species in the sample fluid may be polarized, and the electrical polarization of the biological species will result in them being pushed away from the high field region (in case of negative dielectrophoresis). The electrical field therefore acts as a field barrier and prevents certain biological species from crossing the electrodes. The electrodes establish an electrical field. It is also possible to use more than two electrodes to establish an electrical field, as can also be seen from the Examples. It should also be noted that more than two compartments can be used. For example, it is possible to use three compartments being separated by two membranes.

In a preferred embodiment, the electrical field is established close to the membrane in the compartment containing the sample fluid.

In another preferred embodiment, the compartments may have an inlet and an outlet for the fluids allowing a flow of the sample fluid and the receiving fluid, respectively, through the compartments.

In a preferred embodiment, the electrodes are Pt- or Au-electrodes. The electrodes may be arranged in parallel lines, or on a separate wall close to the membrane openings. Typically, the voltages applied are in the range of 0.5 to 20 V at a frequency in the range of 1 kHz to 50 MHz, preferably 10 kHz to 10 MHz.

In practice, different biological species, like different cell types, have a different polarizability. It is therefore possible to use this property to separate different biological species. For example, the Clausius-Mossotti factor, which can be used to calculate or estimate the dielectrophoretic force on a bioparticle, and thus the necessary electrical field barrier, is different for different cell types. When this is taken into account then the electrical field barrier is e.g. approximately a factor of 100 times higher for human cells than for bacteria.

By tuning the voltage or frequency of the E-field applied to the electrodes it is possible to create an electrical obstruction for e.g. human cells which is still sufficiently weak for e.g. bacteria to cross. This helps to discriminate bacteria from human cells but also offers another advantage as it reduces the contact between the pores and the human cells, and therefore reduces the chance of clogging of the device by keeping larger cells away from the pores. The conductivity of the receiving fluid can also be tuned to maximize e.g. the barrier effect for human cells while minimizing it for bacteria.

The viability of biological material affects the ability of the biological material to show chemotaxis. The more viable a biological material species is, the more chemotaxis it may show. It is thus possible to separate more viable species of a biological material from other less viable species. This effect can even be enhanced using the dielectrophoretic response. The dielectrophoretic response of bacteria changes when the viability of the bacteria is changed e.g. via antibiotics. It is therefore also possible to incorporate an electrical field were the electrical field barrier is tuned so that the bacteria that can enter the receiving fluid are discriminated on the basis of viability.

The above described method is particularly suitable for integration in microfluidic devices, like lab-on-chip devices or in sample-transfer devices. Such devices can be used for the method of the present invention. Furthermore, when separating e.g. bacterial cells from white blood cells, the 'background' (human) DNA is largely reduced which also favors the performance of a genetic test: Background DNA competes with bacterial DNA for binding to silica surfaces and can lead to non-specific amplification in a PCR reaction.

In a preferred embodiment, the sample flow is in the range of 1 to 500 µl/min, preferably in the range of 10 to 100 µl/min, more preferably in the range of 10 to 50 µl/min. Preferably, the device has a length of 1 to 100 mm, and the flow chamber has a width and height of 20 to 500 µm. The membrane thickness, corresponding to the chemotaxis distance, may be in the range of 10 to 200 µm.

In the following, the invention will be illustrated with non-limiting Examples. These examples should not be considered as limiting the present invention.

### EXAMPLES

### Example 1

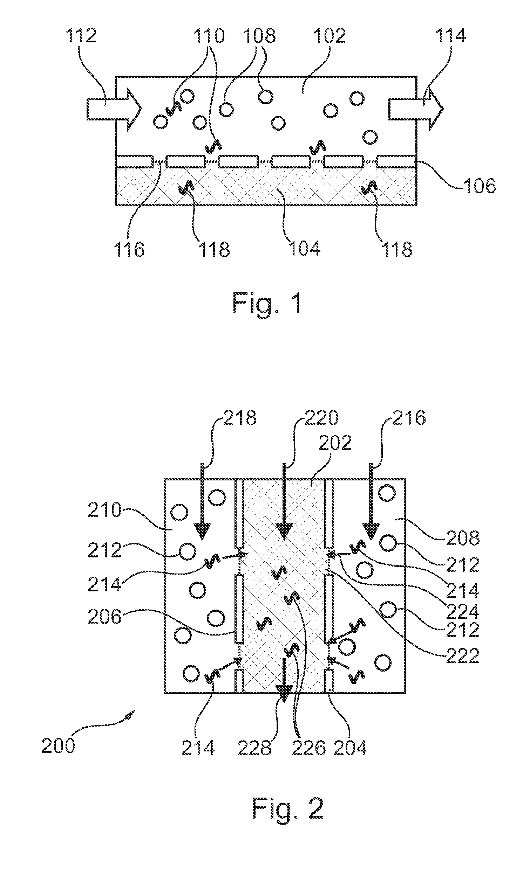

Fig. 1 shows a first embodiment of the present invention. Two volumes 102 and 104 are separated by a porous membrane 106. Said first volume 102 is filled with a sample fluid containing human cells 108 and bacteria 110. Said sample fluid can enter the first volume 102 via an inlet 112, flow along membrane 106 and exit the first volume 102 via an outlet 114. A receiving fluid in the second volume 104 can be in a stationary state or flowing along membrane 106. A growth factor as chemoattractant contained in the receiving fluid can diffuse through pores 116 in said membrane 106 into the sample fluid, thus building a concentration gradient. Said pores 116 are randomly distributed on said membrane 106 and have an average size of 5 µm. A chemotactic reaction is induced in said bacteria 110 drawing these bacteria 110 towards the second volume 104. Said bacteria 110 can pass through said pores 116 into the receiving fluid and are thus separated from the sample fluid. As a chemotactic reaction is not induced in said human cells 108, these human cells 108 will not move towards the receiving fluid, and will not pass said membrane 106. Separated bacteria 118 in the receiving fluid can be collected from the receiving fluid.

### Example 2

In a second Example as illustrated in Fig. 2, a central volume 202 in a microfluidic device 200 is separated by two porous membranes 204 and 206 from two outer volumes 208 and 210. A sample fluid containing human cells 212 and bacteria 214 flows through said outer volumes 208 and 210 along said membranes 204 and 206. The sample fluid can enter said outer volumes 208 and 210 through inlets 216 and 218. A receiving fluid containing a chemoattractant can enter said central volume 202 via an inlet 220 and has a flow direction parallel to the flow direction of the sample fluid. Said bacteria 214 are chemotactically attracted by the chemoattractant and move towards pores 222 in membranes 204 and 206 as indicated by arrow 224. Said membranes 204 and 206 can be made of SU8 or an injection molded plastic plate including either the cover or bottom substrate together with the filter. Separated bacteria 226 can be collected at an outlet 228 of said central volume 202.

### Example 3

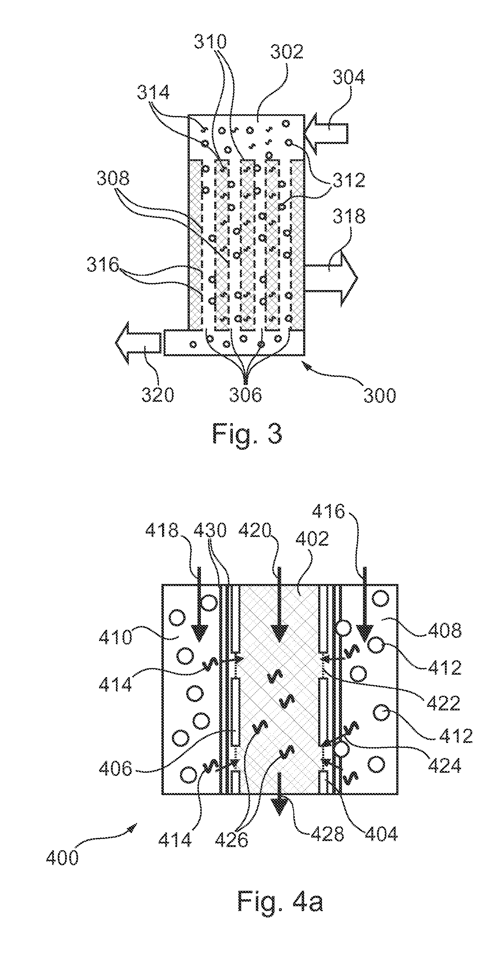

The volume processed by a single device can be increased with multiple channels. Fig. 3 shows a similar setup to Example 2. A sample fluid enters a first volume 302 of a microfluidic device 300 via an inlet 304. Multiple sample fluid channels 306 extend from the first volume, being separated by membranes 308 from multiple receiving fluid channels 310. Said sample fluid containing human cells 312 and bacteria 314 has a contact area with a receiving fluid via membranes 308 separating said sample fluid from said receiving fluid. Multiple receiving fluid channels 310 containing said receiving fluid represent a second volume. A chemoattractant contained in the receiving fluid can diffuse via said membrane 308 into said sample fluid inducing chemotaxis in said bacteria 314. Said bacteria 314 are then drawn towards said membrane 308 and enter said receiving fluid via pores 316 in said membrane 308. The receiving fluid containing said bacteria 314 can be collected at an outlet 318 for said receiving fluid, while the bacteria depleted sample fluid can be collected at an outlet 320 for said sample fluid.

### Example 4

The separating effect achieved by the chemotaxis of the biological material can be increased using electrodes close to the contact area. In a similar device as in Example 2, Fig. 4a shows a setup wherein additional electrodes are placed in a microfluidic device 400 close to the contact area. Again, a central volume 402 is separated by two porous membranes 404 and 406 from two outer volumes 408 and 410. A sample fluid containing human cells 412 and bacteria 414 flows through said outer volumes 408 and 410 along said membranes 404 and 406. The sample fluid can enter said outer volumes 408 and 410 through inlets 416 and 418. A receiving fluid containing a chemoattractant can enter said central volume 402 via an inlet 420 and has a flow direction parallel to the flow direction of the sample fluid. Said bacteria 414 are chemotactically attracted by the chemoattractant and move towards pores 422 in membranes 404 and 406 as indicated by arrow 424. Separated bacteria 426 can be collected at an outlet 428 of said central volume 402. An AC voltage is applied to electrodes 430 placed close to said membranes 404 and 406. The electrodes 430 help to discriminate human cells 412 from bacteria 414. Alternatively more elaborate diagonal electrodes can be extend into membranes 404 and 406 to form barriers to guide bacteria towards the membrane. It is also preferable that identical electrodes are on both the top and bottom plates of the chamber with opposing electrodes being driven with out of phase AC voltages.

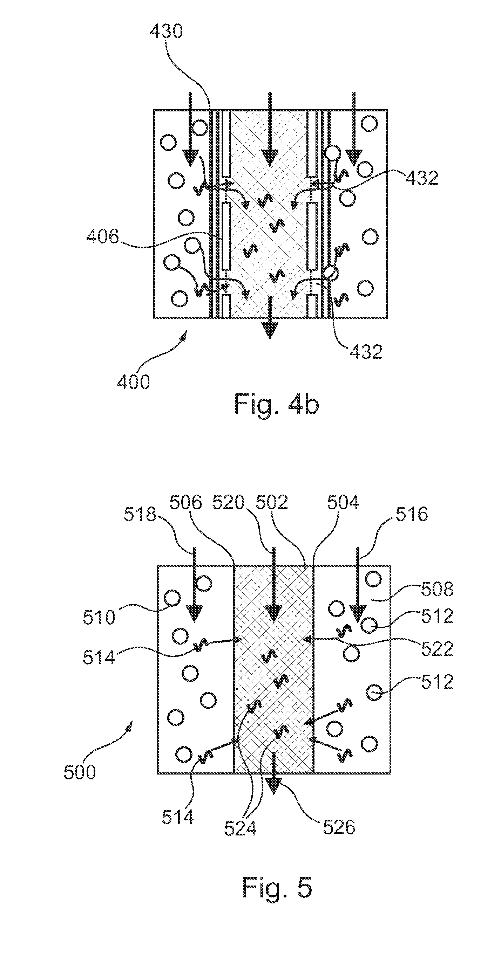

In a preferred embodiment as shown in Fig. 4b, the pressure of said central volume 402 containing said receiving fluid can be lower than the pressure in said outer volumes 408 and 410. With said pressure difference, the sample fluid is drawn through said electrodes 430 and said membranes 406 into said central volume 402 as indicated by arrows 432. The pressure difference helps in pulling said human cells 412 and said bacteria 414 towards said membranes 406. While said human cells 412 are pushed back by an electric field generated by said electrodes 430, said bacteria 414 are influenced less by said electric field and are chemotactically drawn through said membranes 406 into said central volume 402. The differentiation between said human cells 412 and said bacteria 414 is increased.

### Example 5

The separation of two volumes can also be achieved by a laminar flow of the respective fluids. As shown in Fig. 5, a central volume 502 is separated by two direct contact areas 504 and 506 from two outer volumes 508 and 510 in a microfluidic device 500. A sample fluid containing human cells 512 and bacteria 514 flows through said outer volumes 508 and 510 along said direct contact areas 504 and 506. Said contact areas are formed by the laminar flow of the sample fluid in said outer volumes 508 and 510 and the laminar flow of the receiving fluid in said central volume 502. The volumes are in direct contact with each other, however, a substantial mixing of the fluids doesn't occur. The sample fluid can enter said outer volumes 508 and 510 through inlets 516 and 518. A receiving fluid containing a chemoattractant can enter said central volume 502 via an inlet 520 and has a flow direction parallel to the flow direction of the sample fluid. Said bacteria 514 are chemotactically attracted by the chemoattractant and move towards said contact areas 504 and 506 as indicated by arrow 522. Separated bacteria 524 can be collected at an outlet 526 of said central volume 502.

### LIST OF REFERENCE SIGNS

- 102: first volume
- 104: second volume
- 106: porous membrane
- 108: human cells
- 110: bacteria
- 112: inlet for first volume
- 114: outlet for first volume
- 116: pore in membrane
- 118: separated bacteria
- 200: microfluidic device
- 202: central volume
- 204, 206: porous membrane
- 208,210: outer volume
- 212: human cell
- 214: bacteria
- 216, 218: inlets for outer volume
- 220: inlet for central volume
- 222: pore in membrane
- 224: direction of bacteria
- 226: separated bacteria
- 228: outlet for central volume
- 300: microfluidic device
- 302: first volume
- 304: inlet for first volume
- 306: sample fluid channel
- 308: membrane
- 310: receiving fluid channel
- 312: human cell
- 314: bacteria

- 316: pore
- 318: outlet for receiving fluid
- 320: outlet for sample fluid
- 400: microfluidic device
- 402: central volume
- 404, 406: porous membrane
- 408,410: outer volume
- 412: human cell
- 414: bacteria
- 416, 418: inlets for outer volume
- 420: inlet for central volume
- 422: pore in membrane
- 424: direction of bacteria
- 426: separated bacteria
- 428: outlet for central volume
- 430: electrode
- 432: arrows indicating pressure difference
- 500: microfluidic device
- 502: central volume
- 504, 506: direct contact area
- 508,510: outer volume
- 512: human cell
- 514: bacteria
- 516, 518: inlets for outer volume
- 520: inlet for central volume
- 522: direction of bacteria
- 524: separated bacteria
- 526: outlet for central volume

## Claims

1. A method for the separation of biological material (110;214;314;414;514) from a sample fluid comprising the steps of
- providing a sample fluid, wherein the sample fluid contains biological material (110;214;314;414;514);
- providing a receiving fluid;
- allowing an exchange between the sample fluid and the receiving fluid at a contact area (106;204,206;308;404,406;504,506); and
- collecting the receiving fluid containing separated biological material (110;214;314;414;514),
wherein the sample fluid and/or the receiving fluid contains a chemotactic substance inducing chemotaxis in the biological material (110;214;314;414;514).

2. Method according to claim 1, wherein the contact area between the sample fluid and the receiving fluid is a porous membrane (106;204,206;308;404,406).

3. Method according to claim 2, wherein the porous membrane (106;204,206;308;404,406) is permeable to the biological material (110;214;314;414;514).

4. Method according to claim 1, wherein the sample fluid and the receiving fluid are in direct contact (504,506) with each other.

5. Method according to any of the previous claims, wherein the biological material (110;214;314;414;514) is selected from the group consisting of eukaryotic cells, archea, bacteria, fungi, algae, and protozoa, or mammalian cells, preferably human cells.

6. Method according to any of the previous claims, wherein the sample fluid contains a chemotactic substance, and the chemotactic substance is a chemorepellent.

7. Method according to any of the previous claims, wherein the receiving fluid contains a chemotactic substance, and said chemotactic substance is a chemoattractant.

8. Method according to any of the previous claims, wherein the chemotactic substance is selected from the group consisting of sugars, growth factors, chemokines, phenol and phenol derivatives, and alcohols and polyhydric alcohols.

9. Method according to any of the previous claims, wherein at least one of the sample fluid and the receiving fluid is flowing as a continuous flow.

10. Method according to any of the previous claims 9, wherein the flow of the sample fluid and/or the flow of the receiving fluid is substantially laminar.

11. Method according to any of the previous claims, wherein at least one electrode (530) is provided at the contact area or close to the contact area (106;204,206;308;404,406;504,506).

12. Method according to any of the previous claims, wherein the pressure of the sample fluid differs from the pressure in the receiving fluid.

13. Use of a microfluidic device (200;300;400;500) for the separation of biological material (110;214;314;414;514) from a sample fluid.

14. Use according to claim 13, wherein the separation is performed by chemotaxis.

15. Device for the separation of biological material comprising:
- at least two compartments;
- at least one membrane separating said at least two compartments;
- at least two electrodes being located close to the at least one membrane; and optionally one pre-exisiting chemical gradient.
